Europäisches Patentamt

European Patent Office

Office européen des brevets

(19)

(11) Publication number: **0 138 283**
**B1**

(12) **EUROPEAN PATENT SPECIFICATION**

(45) Date of publication of patent specification: **30.03.88**

(51) Int. Cl.⁴: **C 07 D 499/00**

(21) Application number: **84201515.8**

(22) Date of filing: **18.10.84**

(54) **Process for the preparation of 6,6-dibromopenicillanic acid 1,1-dioxide.**

(30) Priority: **18.10.83 EP 83201496**

(43) Date of publication of application:
**24.04.85 Bulletin 85/17**

(45) Publication of the grant of the patent:
**30.03.88 Bulletin 88/13**

(84) Designated Contracting States:
**AT BE CH DE FR GB IT LI LU NL SE**

(56) References cited:
**EP-A-0 073 674**
**EP-A-0 093 465**
**GB-A-2 045 755**

(73) Proprietor: **Gist - Brocades N.V.**
**Wateringseweg 1 P.O. Box 1**
**NL-2600 MA Delft (NL)**

(72) Inventor: **Kapur, Jagdish Chander**
**Roland Holstlaan 799**
**NL-2624 KB Delft (NL)**
Inventor: **Fasel, Herman Pieter**
**Van der Haertstraat 19**
**NL-2613 XZ Delft (NL)**

(74) Representative: **Huygens, Arthur Victor, Dr.**
**et al**
**c/o Gist-Brocades N.V. Patent & Trademarks**
**Department Wateringseweg 1 PO Box 1**
**NL-2600 MA Delft (NL)**

Courier Press, Leamington Spa, England.

**0 138 283**

**Description**

The invention relates to a process for the preparation of 6,6-dibromopenicillanic acid 1,1-dioxide by reaction of 6-beta-aminopenicillanic acid 1,1-dioxide with a nitrosating agent in the presence of an inorganic or strong organic acid and bromine.

The presumed association between the resistance of certain bacteria to beta-lactam antibiotics and the capability of these bacteria to produce and secrete beta-lactamases has led to an intensive search for beta-lactamase inhibitors.

It is known from Dutch patent application 7806126 that penicillanic acid 1,1-dioxide and salts and esters thereof, have useful pharmacological properties, for example as effective inhibitors of several types of beta-lactamases present in various kinds of bacteria. In the beforementioned Dutch application a process is described for the preparation of penicillanic acid 1,1-dioxide and salts and esters thereof by oxidation of penicillanic acid.

Another process for the preparation of penicillanic acid 1,1-dioxide is described in Dutch patent application 8001285. In this application penicillanic acid 1,1-dioxide is prepared by diazotisation-bromination of 6-amino-penicillanic acid followed by oxidation of the formed 6,6-dibromopenicillanic acid into 6,6-dibromo-penicillanic acid 1,1-dioxide and dehalogenation of the latter compound.

In European patent application 83200541, publication No. 0093465, a process is described for the preparation of a mixture of 6,6-dibromopenicillanic acid 1,1-dioxide and 6-alpha-bromo penicillanic acid 1,1-dioxide by diazotisation-bromination of 6-aminopenicillanic acid 1,1-dioxide. The relative amount of dibromo compound in the mixture usually varies from 80—90%, the relative amount of monobromo compound from 10—20%. The mixture of the bromocompounds thus prepared can be reduced into penicillanic acid 1,1-dioxide for instance in the way as described in European patent application 83200542, publication No. 0092286.

In the above mentioned European patent application 83200541, publication No. 0093465, the mixture of 6,6-dibromopenicillanic acid 1,1-dioxide and 6-alpha-bromopenicillanic acid 1,1-dioxide is prepared by reaction of 6-aminopenicillanic acid 1,1-dioxide with a nitrosating agent in the presence of hydrogen bromide and bromine. The reaction is performed at a temperature between −20°C and 30°C with at least an equimolar amount of a nitrosating agent in the presence of 1 to 5 equivalents of a strong inorganic or organic acid in solution or suspension of a mixture of water and a partly or completely water-miscible organic solvent medium, the amount of water present being from 1 to 20% by volume, containing hydrogen bromide and bromine in at least equimolar amounts. Optionally an auxiliary agent which facilitates the bromination of the diazotized intermediate in an amount varying from 10% to at least an equimolar amount of the aminopenicillanic acid 1,1-dioxide starting material can be used. The maximum yield of 6,6-dibromopenicillanic acid 1,1-dioxide and 6-alpha-bromopenicillanic acid 1,1-dioxide in the examples of the above mentioned European patent application calculated on the starting 6-aminopencillanic acid 1,1-dioxide did not exceed the 70%, in spite of the extensive disclosure.

It has now been found that pure 6,6-dibromo-penicillanic acid 1,1-dioxide can be prepared in a considerably higher yield than described in the abovementioned European patent application 83200541, publication No. 0093465, by the addition of a small amount of an alcohol to the reaction mixture of the diazotisation-bromination reaction of 6-aminopencillanic acid 1,1-dioxide. The present invention, therefore, relates to a process for the preparation of 6,6-dibromopenicillanic acid 1,1-dioxide by reaction of 6-beta-aminopenicillanic acid 1,1-dioxide with a nitrosating agent in the presence of an inorganic or strong organic acid and bromine, characterized in that an alcohol is present in the reaction mixture.

When using the process of the present invention it is possible to prepare pure 6,6-dibromopenicillanic acid 1,1-dioxide in a yield of 90%. In comparison with the process described in European patent application 83200541, publication No. 0093465, a relative improvement of the amount of bromo compounds of about 30% is reached. This means that the cost price of the bromo compounds as prepared by the older process will be 30% higher than the cost price of the dibromo compound as prepared by the process of the present invention. The improvement of the yield is illustrated for instance by Example 3 and Comparative Example 1 of the present invention. Keeping all the other reaction conditions the same, the yield of 6,6-dibromopenicillanic acid 1,1-dioxide improves from 53 to 90% by the addition of a small amount of methanol.

Furthermore the recovery procedure in the present process is usually more simple than in the process described in the beforementioned European patent application 83200541, publication No. 0093465. In that application it is necessary to remove the water-miscible solvent by azeotropic distillation or by evaporation *in vacuo*, as otherwise the extraction of the product from the aqueous layer would give problems. As the reaction of the present invention can be performed in water immiscible solvents, for example methylene chloride it is no longer necessary to remove the water-miscible solvent, which is an important feature in a large-scale chemical process.

It is another advantage of the present application that 6,6-dibromopenicillanic acid 1,1-dioxide of a high purity is obtained. The ultimately desired product, i.e. penicillanic acid 1,1-dioxide, will therefore also have a high purity, which is an important feature of compounds to be used for the preparation of pharmaceutical compositions. Also the fact that the process of the present invention results in pure

2

6,6-dibromo penicillanic acid 1,1-dioxide and is not mixed with an amount of 6-alpha-bromo-penicillanic acid 1,1-dioxide has a positive influence on the quality of the end product.

The 6,6-dibromo penicillanic acid 1,1-dioxide can be converted into the desired acid 1,1-dioxide according to known methods, as for instance described in British patent application 2045755. Also the process as described in European patent application 83200542, publication No. 0092286, can be used.

The reaction is carried out by addition of bromine, aqueous hydrobromic acid and the alcohol to a solution or suspension of 6-aminopenicillanic acid 1,1-dioxide in an inert organic solvent followed by the addition of the nitrosating agent. After stirring for 5 minutes to 1 hour any excess of bromine is removed, for instance by addition of an aqueous solution of sodium bisulfite. Extraction with an organic solvent, drying in the usual way and evaporation of the solvent results in the desired 6,6-dibromopenicillanic acid 1,1-dioxide.

Examples of alcohols which can be used in the process of the present invention are methanol, ethanol, propanol, i-propanol, t-butylalcohol, pentanol, hexanol, cyclohexanol, benzylalcohol, 1,2-propanediol and glycerol. However, the scope of the present invention is not limited to the above mentioned alcohols, but can also be extended to other alcohols. Preferably an alkanol or alkanediol having 1 to 4 carbon atoms, more preferably methanol is used. The amount of alcohol may vary between 1 and 10 equivalents calculated on the starting 6-aminopenicillanic acid 1,1-dioxide.

Preferably 2—6 equivalents of the alcohol are used, more preferably 4 equivalents. Examples of inorganic and strong organic acids are hydrobromic acid, hydrochloric acid, sulphuric acid, phosphoric acid, aminosulphonic acid, chloroacetic acid, dichloroacetic and trifluoroacetic acid. Preferably hydrobromic acid is used.

Suitable inert organic solvents in which the reaction can be carried out are, for example methylene chloride, chloroform and acetonitrile. Preferably methylene chloride is used.

The reaction can be carried out at a temperature between −20° and 30°C, preferably between −10 and 15°C.

The nitrosating agent to be used in the reaction can be an alkali metal nitrite or an alkylnitrite. Preferably sodium nitrite is used.

The starting 6-aminopenicillanic acid 1,1-dioxide can be prepared as described in European Patent 0030771.

The following non-limitative examples illustrate the present invention.

General remarks
1. Alcohol-free dichloromethane has been used in all the examples.
2. The purity of 6,6-dibromopenicillanic acid 1,1-dioxide has been determined through 60 MHz-NMR spectra using either maleic acid as the reference in dimethylsulfoxide-$d_6$ or 2,6-dichloroacetophenone as the reference in acetone-$d_6$, unless otherwise stated.

Example 1
To a suspension of 6-beta-aminopenicillanic acid 1,1-dioxide (6.2 g; purity by HPLC 90%; 22.5 mmol) in dichloromethane (75 ml) cooled to about −5° to −0°C was added a solution of bromine (6.0 g; 37.5 mmol) in dichloromethane (25 ml), hydrobromic acid (7.1 ml; 64.0 mmol) and methanol (2 ml; 49.4 mmol). Sodium nitrite (2.05 g; 29.7 mmol) was added portionwise over a period of 10—15 min. During the addition of sodium nitrite (2.05 g; 29.7 mmol) the reaction mixture was maintained at 0° to 5°C. The contents were further stirred for 30 min at the same temperature. A solution of sodium bisulfite (35 ml; 10% aqueous) was added dropwise at 0° to 5°C till the bromine colour was discharged. The reaction mixture was extracted with chloroform (4×200 ml). The combined extracts were washed with brine (2×100 ml) and dried over anhydrous magnesium sulfate. Removal of the solvent, washings with n-hexane and drying under reduced pressure resulted in 7.29 g of 6,6-dibromopenicillanic acid 1,1-dioxide. IR (KBr): 2700—3300, 1811, 1740, 1333 cm$^{-1}$; NMR (DMSO-$d_6$, delta-values in ppm, TMS, 60 MHz); 1.43 (s, 3H, CH$_3$), 1.53 (s, 3H, CH$_3$), 4.72 (s, 1H, C$^3$H), 6.02 (s, 1H, C$^5$H). Purity 99.1%, thus giving a yield of 82.2%. The purity was determined through 60 MHz NMR spectroscopy using maleic acid as the reference.

Example 2
The reaction was carried out as described in Example 1, however, the amount of methanol used was 3 ml (74.1 mmol) instead of 2 ml. Isolated yield of 6,6-dibromopenicillanic acid 1,1-dioxide=7.93 g (purity 98.5%), thus giving a yield of 88.9%.

Example 3
The reaction was carried out as described in Example 1, however, the amount of methanol used was 4 ml (98.75 mmol) instead of 2 ml. Isolated yield of 6,6-dibromopenicillanic acid 1,1-dioxide=7.98 g (purity 99.1%), thus giving a yield of 89.9%.

Example 4
The reaction was carried out as described in Example 1, however, the amount of methanol used was 5 ml (123.4 mmol) instead of 2 ml. Isolated yield of 6,6-dibromopenicillanic acid 1,1-dioxide=8.09 g (purity 90.9%), thus giving a yield of 83.6%.

## Example 5

The reaction was carried out as described in Example 1, however, the amount of methanol used was 6 ml (148.1 mmol) instead of 2 ml. Isolated yield of 6,6-dibromopenicillanic acid 1,1-dioxide=8.23 g (purity 92.6%), thus giving a yield of 86.7%.

## Example 6

The reaction was carried out as described in Example 1, however, the amount of hydrobromic acid was 7.46 ml (67 mmol). The amount of methanol used was 1 ml (24.7 mmol). Isolated yield of 6,6-dibromopenicillanic acid 1,1-dioxide=7.08 g (purity 99.4%), thus giving a yield of 80.1%.

## Example 7

The reaction was carried out as described in Example 6, however, the amount of methanol used was 2 ml (49.4 mmol). Isolated yield of 6,6-dibromopenicillanic acid 1,1-dioxide=7.72 g (purity 96.9%), thus giving a yield of 85.2%.

## Example 8

The reaction was carried out as described in Example 1, however, the amount of methanol used was 3 ml (74.06 mmol). Isolated yield of 6,6-dibromopenicillanic acid 1,1-dioxide=7.54 g (purity 98.6%), thus giving a yield of 84.6%.

## Example 9

The reaction was carried out as described in Example 6, however, the amount of methanol used was 4 ml (98.8 mmol). Isolated yield of 6,6-dibromopenicillanic acid 1,1-dioxide=8.00 g (purity 96.97%), thus giving a yield of 88.3%.

## Example 10

The reaction was carried out as described in Example 6, however, the amount of methanol used was 5 ml (123.4 mmol). Isolated yield of 6,6-dibromopenicillanic acid 1,1-dioxide=8.17 g (purity 95.7%), thus giving a yield of 89%.

## Example 11

The reaction was carried out as described in Example 6, however, the amount of methanol used was 6 ml (148.1 mmol). Isolated yield of 6,6-dibromopenicillanic acid 1,1-dioxide=8.01 g (purity 97.7%), thus giving a yield of 89%.

## Example 12

The reaction was carried out as described in Example 1, however, the amount of methanol used was 7 ml (172.8 mmol). Isolated yield of 6,6-dibromopenicillanic acid 1,1-dioxide=7.10 g (purity 94.3%), thus giving a yield of 77%. The mother liquor from the isolated product was concentrated in vacuum and dried. NMR analyses of the residue showed that it contained 0.4 g of 6,6-dibromopenicillanic acid 1,1-dioxde=5.3%, thus giving a total yield of 82.3%.

## Example 13

The reaction was carried out as described in Example 1, however, the amount of HBr used was 7.83 ml (70.4 mmol). Isolated yield of 6,6-dibromopenicillanic acid 1,1-dioxide=7.76 g (purity 98.1%), thus giving a yield of 86.6%.

## Example 14

The reaction was carried out as described in Example 13, however, the amount of methanol used was 3 ml (74.1 mmol). Isolated yield of 6,6-dibromopenicillanic acid 1,1-dioxide=7.73 g (purity 93.75%), thus giving a yield of 82.5%.

## Example 15

The reaction was carried out as described in Example 13, however, the amount of methanol used was 4 ml (98.8 mmol). Isolated yield of 6,6-dibromopenicillanic acid 1,1-dioxide=7.96 g (purity 99.8%), thus giving a yield of 90.3%.

## Example 16

The reaction was carried out as described in Example 13, however, the amount of methanol used was 5 ml (123.4 mmol). Isolated yield of 6,6-dibromopenicillanic acid 1,1-dioxide=8.01 g (purity 96.65%), thus giving a yield of 88.1%.

**Example 17**

The reaction was carried out as described in Example 13, however, the amount of methanol used was 6 ml (148.13 mmol). Isolated yield of 6,6-dibromopenicillanic acid 1,1-dioxide=8.09 g (purity 96.9%), thus giving a yield of 89.1%.

**Example 18**

The reaction was carried out as described in Example 1, however, the amount of methanol used was 4 ml (98.8 mmol). The amount of hydrobromic acid was 2 ml (17.96 mmol). Isolated yield of 6,6-dibromopenicillanic acid 1,1-dioxide=7.43 g (purity 87.95%), thus giving a yield of 75.2%.

**Example 19**

The reaction was carried out as described in Example 18, however, the amount of hydrobromic acid was 3 ml (26.94 mmol). Isolated yield of 6,6-dibromopenicillanic acid 1,1-dioxide=7.63 g (purity 89.55%), thus giving a yield of 78.6%.

**Example 20**

The reaction was carried out as described in Example 18, however, the amount of hydrobromic acid was 4.63 ml (41.56 mmol). Isolated yield of 6,6-dibromopenicillanic acid 1,1-dioxide=7.81 g (purity 93.95%), thus giving a yield of 84.4%.

**Example 21**

The reaction was carried out as described in Example 18, however, the amount of hydrobromic acid was 5.3 ml (47.56 mmol). Isolated yield of 6,6-dibromopenicillanic acid 1,1-dioxide=7.77 g (purity 95.7%), thus giving a yield of 85.6%.

**Example 22**

The reaction was carried out as described in Example 18, however, the amount of hydrobromic acid was 6.4 ml (57.56 mmol). Isolated yield of 6,6-dibromopenicillanic acid 1,1-dioxide=7.84 g (purity 95.2%), thus giving a yield of 85%.

**Example 23**

The reaction was carried out as described in Example 18, however, the amount of hydrobromic acid was 9.17 ml (82.35 mmol). Isolated yield of 6,6-dibromopenicillanic acid 1,1-dioxide=7.79 g (purity 97.35%), thus giving a yield of 86.1%.

**Example 24**

Performed as Example 1 using 6-beta-aminopenicillanic acid 1,1-dioxide (31 g; purity by HPLC=90%; 112.5 mmol), dichloromethane (375 ml), bromine (30 g; 187.5 mmol) in dichloromethane (125 ml), hydrobromic acid (35.4 ml; 318 mmol), methanol (20 ml; 493.8 mmol) sodium nitrite (10.25 g; 148.5 mmol), sodium metabisulfite (18 g in 180 ml water) and brine (2×500 ml). Isolated yield of 6,6-dibromopenicillanic acid 1,1-dioxide 39.65 g (purity 99.3%), thus giving a yield of 89.6%.

**Example 25**

The reaction was carried out as described in Example 1, however, the amount of methanol used was 4 ml (98.75 mmol), the amount of hydrobromic acid was 7.46 ml (67 mmol) and pentylnitrite, 3.95 ml (29.7 mmol), was used instead of sodium nitrite. Isolated yield of 6,6-dibromopenicillanic acid 1,1-dioxide=7.0 g (purity=93.75%), thus giving a yield of 75.5%.

**Example 26**

The reaction was carried out as described in Example 9, however, the amount of 6-beta-aminopenicillanic acid 1,1-dioxide used was 6.2 g (purity by HPLC=88%; 22 mmol) and methanesulfonic acid (7.5 ml; 67 mmol) was used instead of hydrobromic acid (7.46 ml; 67.0 mmol). Isolated yield=1.06 g.

**Example 27**

The reaction was carried out as described in Example 26, however, using $H_3BO_3$ (4.14 g; 67 mmol) instead of methanesulfonic acid. Isolated yield=3.65 g.

**Example 28**

The reaction was carried out as described in Example 26, however, using phosphoric acid (7.5 ml; 67 mmol) instead of methanesulfonic acid. Isolated yield=5.98 g.

**Example 29**

The reaction was carried out as described in Example 26, however, using sulphuric acid (7.5 ml; 67 mmol) instead of methanesulfonic acid. Isolated yield=2.97 g.

### Example 30
The reaction was carried out as described in Example 9, however, with 6-beta-aminopenicillanic acid 1,1-dioxide (6.2 g; purity by HPLC=88%; 22 mmol) and chloroform instead of dichloromethane as solvent. Isolated yield=7.15 g.

### Example 31
The reaction was carried out as described in Example 30, however, using ethyl acetate instead of chloroform as solvent. Isolated yield=5.68 g, which contained about 6% of 6-alpha-bromopenicillanic acid 1,1-dioxide. The mother-liquor after isolating the product contained (through HPLC) 6.5% and 3.7% of 6,6-dibromopenicillanic acid 1,1-dioxide and 6-alpha-bromo-penicillanic acid 1,1-dioxide respectively from a total weight of 1.52 g after evaporating the solvent.

### Example 32
The reaction was carried out as described in Example 30, however, using acetonitrile instead of chloroform as solvent. Isolated yield=7.15 g, which contained about 4% of 6-alpha-bromopenicillanic acid 1,1-dioxide (through HPLC).

### Example 33
The reaction was carried out as described in Example 30, however, using tetrahydrofuran instead of chloroform as solvent. Furthermore, pure bromine was added to the reaction mixture (at −5 to −0°C) instead of a solution of bromine in the solvent. Isolated yield=3.17 g, which contained about 3.5% of 6-alpha-bromopenicillanic acid 1,1-dioxide. The mother-liquor after isolating the product also contained 4.1% and 58% respectively of 6-alpha-bromopenicillanic acid 1,1-dioxide and 6,6-dibromopenicillanic acid 1,1-dioxide respectively from a total weight of 1.58 g after evaporating the solvent, (purity determined through HPLC).

### Example 34
The reaction was carried out as described in Example 30, however, using nitromethane instead of chloroform as solvent. Isolated yield=5.75 g, which contained about 1.2% of 6-alpha-bromopenicillanic acid 1,1-dioxide. The mother-liquor after isolating the product contained 1.3% and 41% respectively of 6-alpha-bromopenicillanic acid 1,1-dioxide and 6,6-dibromopenicillanic acid 1,1-dioxide respectively from a total weight of 1.44 g after evaporating the solvent.

### Example 35
The reaction was carried out as described in Example 9, however, with 6-beta-aminopenicillanic acid 1,1-dioxide (6.29 g; purity by HPLC=86.5%; 21.62 mmol) and ethanol (96%; 5.6 ml; 98.9 mmol) instead of methanol. Isolated yield=6.98 g.

### Example 36
The reaction was carried out as described in Example 9, however, the amount of 6-beta-aminopenicillanic acid 1,1-dioxide used was 6.2 g (purity by HPLC=88%; 22 mmol) and n-butanol (9 ml; 98.9 mmol) was used instead of methanol. Isolated yield=6.84 g.

### Example 37
The reaction was carried out as described in Example 36, however, using isopropanol (7.6 ml; 98.9 mmol) instead of n-butanol. Isolated yield=6.66 g.

### Example 38
The reaction was carried out as described in Example 36, however, using isobutanol (9.13 ml; 98.9 mmol) instead of n-butanol. Isolated yield=6.37 g.

### Example 39
The reaction was carried out as described in Example 36, however, using cyclohexanol (10.3 ml; 98.9 mmol) instead of n-butanol. Isolated yield=5.4 g.

### Example 40
The reaction was carried out as described in Example 36, however, using 1,2-propanediol (7.26 ml; 98.9 mmol) instead of n-butanol. Isolated yield=7.02 g.

### Comparative Example 1
The reaction was performed as described in Example 1, however, the amount of hydrobromic acid was 7.46 ml (67 mmol) and no methanol was used. Isolated yield of 6,6-dibromopenicillanic acid 1,1-dioxide=4.72 g (purity 98.8%), thus giving a yield of 53.1%.

6

Comparative Example 2

The reaction was carried out as described in Example 1, however, the amount of hydrobromic acid was 7.46 ml (67 mmol) and methanol (75 ml) was used as solvent instead of methylene chloride. Bromine (5.99 g, 37.5 mmol) was added as solution in methanol (125 ml). After neutralization of excess bromine, the extractions with chloroform were carried out at pH 3.5. Isolated yield of the crude product=5.83 g. The NMR spectrum of the crude product showed the presence of 6,6-dibromopenicillanic acid 1,1-dioxide, 6-alpha-bromopenicillanic acid 1,1-dioxide and 6-bromo-6-methoxypenicillanic acid 1,1-dioxide (molar ratio: 2.8:6.9:9.5 respectively) and cleaved product(s). The amount of these compounds was determined through 60 MHz NMR spectroscopy using maleic acid as the reference (yield=13.05%; 26.95% and 41.05% respectively), thus giving a yield of 6,6-dibromopenicillanic acid 1,1-dioxide=8.7%; 6-alpha-bromopenicillanic acid 1,1-dioxide=22.4% and 6-bromo-6-methoxypenicillanic acid 1,1-dioxide=31.1%. The structure of 6-bromo-6-alpha-methoxypenicillanic acid 1,1-dioxide was confirmed by identification bf the corresponding methyl ester as described below: 3 g of the above crude bromo-acids were taken in ether and cooled to 0°C. Then diazomethane (solution in ether) was added till the yellow colour persisted. The mixture was stirred at the same temperature for 45 min. The excess of diazomethane was destroyed by addition of glacial acetic acid. The solvent was removed under reduced pressure. The resulting product was taken up in ethyl acetate (60 ml), water (10 ml) was added and the solution was cooled to about 0°C. Then with 1N NaOH, the pH was brought to 7.0. The organic layer was separated, washed with brine, dried over anhydrous magnesium sulfate and the solvent was removed under reduced pressure to a thick oily liquid (2.59 g) containing a mixture of the methyl esters of 6,6-dibromopenicillanic acid 1,1-dioxide, 6-alpha-bromopenicillanic acid 1,1-dioxide and 6-bromo-6-alpha-methoxypenicillanic acid 1,1-dioxide as the main products. 2.2 g of this mixture was chromatographed by means of HPLC (Merck Column C) using ethylacetate-$n$-hexane (1:3) as the eluent. The fractions containing the 6-bromo-6-alpha-methoxypenicillanic acid 1,1-dioxide methyl ester were collected and evaporated under reduced pressure to a solid product, yield=0.889 g. IR ($CHCl_3$) 1810, 1760, 1339 $cm^{-1}$. NMR ($CDCl_3$): delta-values in ppm (TMS, 60 MHz) 1.42 (s, 3H), 1.63 (s, 3H), 3.68 (s, 3H), 3.88 (s, 3H), 4.53 (s, 1H), 4.30 (s, 1H). Mass spectrum m/e 355, 357.

## Claims

1. Process for the preparation of 6,6-dibromo-penicillanic acid 1,1-dioxide by reaction of 6-beta-aminopenicillanic acid 1,1-dioxide with a nitrosating agent in an inert, organic solvent in the presence of an inorganic or strong organic acid and bromine, characterized in that the amount of acid is not less than 0.8 equivalent (related to the amount of 6-beta-aminopenicillanic acid) and that 1 to 10 equivalents (related to the amount of 6-beta-aminopenicillanic-acid) of an alcohol are present in the reaction mixture.

2. Process according to claim 1, characterized in that the alcohol is an alkanol or alkanediol having 1 to 4 carbon atoms, preferably methanol.

3. Process according to claim 1—2, characterized in that the amount of methanol is 2—6 equivalents, preferably 4 equivalents.

4. Process according to claims 1—3, characterized in that the nitrosating agent is an alkali metal nitrite, preferably sodium nitrite.

5. Process according to claims 1—3, characterized in that the nitrosating agent is an alkylnitrite.

6. Process according to claims 1—5, characterized in that the inorganic acid is hydrobromic acid.

7. Process according to claims 1—6, characterized in that the reaction is performed in a water immiscible solvent, preferably methylene chloride.

8. Process according to claims 1—7, characterized in that the reaction is carried out at a temperature between −20 and 30°C, preferably between −10 and 15°C.

## Patentansprüche

1. Verfahren zur Herstellung von 6,6-Dibrompenicillansäure-1,1-dioxid durch Umsetzung von 6β-Aminopencillansäure-1,1-dioxid mit einem Nitrosierungsmittel in einem inerten, organischen Lösungsmittel in Gegenwart einer anorganischen oder starken organischen Säure und von Brom, dadurch gekennzeichnet, dass die Menge der Säure nicht geringer als 0,8 Aequivalent (bezogen auf die Menge an 6β-Aminopenicillansäure) ist und dass 1 bis 10 Aequivalente (bezogen auf die Menge an 6β-Aminopenicillansäure) eines Alkohols in dem Reaktionsgemisch vorhanden sind.

2. Verfahren nach Anspruch 1, dadurch gekennzeichnet, dass der Alkohol ein Alkanol oder Alkandiol mit 1 bis 4 Kohlenstoffatomen, vorzugsweise Methanol, ist.

3. Verfahren nach Anspruch 1 bis 2, dadurch gekennzeichnet, dass die Menge an Methanol 2 bis 6 Aequivalente, vorzugsweise 4 Aequivalente, beträgt.

4. Verfahren nach Ansprüchen 1 bis 3, dadurch gekennzeichnet, dass das Nitrosierungsmittel ein Alkalimetallnitrit, vorzugsweise Natriumnitrit, ist.

5. Verfahren nach Ansprüchen 1 bis 3, dadurch gekennzeichnet, dass das Nitrosierungsmittel ein Alkylnitrit ist.

**0 138 283**

6. Verfahren nach Ansprüchen 1 bis 5, dadurch gekennzeichnet, dass die anorganische Säure Bromwasserstoffsäure ist.

7. Verfahren nach Ansprüchen 1 bis 6, dadurch gekennzeichnet, dass die Reaktion in einem mit Wasser nicht mischbaren Lösungsmittel, vorzugsweise Methylenchlorid, ausgeführt wird.

8. Verfahren nach Ansprüchen 1 bis 7, dadurch gekennzeichnet, dass die Reaktion bei einer Temperatur zwischen −20 und 30°C, vorzugsweise zwischen −10 und 15°C, ausgeführt wird.

**Revendications**

1. Procédé de préparation du 1,1-dioxyde d'acide 6,6-dibromopénicillanique par réaction du 1,1-dioxyde d'acide 6-bêta-aminopénicillanique avec un agent de nitrosation dans un solvant organique inerte en présence d'un acide inorganique ou organique fort et de brome, caractérisé en ce que la quantité d'acide n'est pas inférieure à 0,8 équivalent (par rapport à la quantité d'acide 6-bêta-aminopénicillanique) et que 1 à 10 équivalents (par rapport à la quantité d'acide 6-bêta-aminopénicillanique) d'un alcool sont présents dans le mélange de réaction.

2. Procédé suivant la revendication 1, caractérisé en ce que l'alcool est un alcanol ou alcanediol comptant 1 à 4 atomes de carbone, de préférence le méthanol.

3. Procédé suivant les revendications 1 et 2, caractérisé en ce que la quantité de méthanol est de 2 à 6 équivalents, de préférence de 4 équivalents.

4. Procédé suivant les revendications 1 à 3, caractérisé en ce que l'agent de nitrosation est un nitrite de métal alcalin, de préférence le nitrite de sodium.

5. Procédé suivant les revendications 1 à 3, caractérisé en ce que l'agent de nitrosation est un nitrite d'alcoyle.

6. Procédé suivant les revendications 1 à 5, caractérisé en ce que l'acide inorganique est l'acide bromhydrique.

7. Procédé suivant les revendications 1 à 6, caractérisé en ce que la réaction est exécutée dans un solvant non miscible à l'eau, de préférence le chlorure de méthylène.

8. Procédé suivant les revendications 1 à 7, caractérisé en ce que la réaction est exécutée à une température située entre −20 et 30°C, de préférence entre −10 et 15°C.